# EUROPEAN PATENT APPLICATION

(11) **EP 2 899 261 A1**
(43) Date of publication of application: **29.07.2015**
(21) Application number: 15152543.3
(22) Date of filing: 26.01.2015
(51) Int. Cl.: C12M 1/00, C12M 1/02

(54) **Arrangement of a photobioreactor or a microbiological reactor**

(30) Priority: 28.01.2014 NL 2012157
(71) Applicant: Photanol B.V., 1018 WB Amsterdam (NL)
(72) Inventor: Hellingwerf, Klaas Jan, 1098 WS Amsterdam (NL); van Lieshout, Theo, 1018 WB Amsterdam (NL); Koblens, Paul, 1551 GB Westzaan (NH) (NL); van Grondelle, Wilmar, 1057 ZZ Amsterdam (NL); van Alphen, Pascal, 1445 HA Purmerend (NL)
(74) Representative: Nederlandsch Octrooibureau

(57) **Abstract**

A reactor for biochemical and/or photochemical processes includes a tank having a volume for containing a reaction fluid, a heat transfer unit being arranged in the volume, a plurality of light units arranged in the volume of the tank. The heat transfer unit comprises a plurality of heat pipes. The light units are mounted on the plurality of heat pipes.

## Description

### Field of the invention

The present invention relates to a reactor for biochemical processes comprising a tank having a volume for containing a reaction fluid, a heat transfer unit being arranged in the volume, a plurality of light units arranged in the volume of the tank. Moreover, the present invention also relates to the use of the reactor.

### Background of the invention

In general, a reactor for biochemical processes having a tank for containing a fluid, with a heat transfer unit and lights arranged in the volume of the tank are known in the art.

In particular, photo-bioreactors are known that contain a reaction fluid in a form of an aqueous solution with phototrophic microorganisms that are cultivated by utilizing light energy. These organisms use the process of photosynthesis to build their own biomass from light, water and carbon dioxide (CO₂). In a similar manner in microbiological reactors a fluid containing microorganisms, often obtained after selected molecular genetic engineering, is exposed to light and usually nutritive substances to have the microorganisms convert these into a reaction product. prior art reactor designs prevented the photo-bioreactor to be operated at sufficient light intensity to guarantee operation of the microorganism 'close to light saturation' at high cell densities (> 1 g dwt/1).

In WO 2010/115655 a photo-bioreactor is disclosed which comprises a plurality of light arrays arranged inside the volume of a container. The light arrays are positioned equi-distantly with respect to each other to provide homogeneously distributed illumination of the volume inside the container. Each of the light arrays is connected to a computer-controlled power supply that regulates the power supplied to the light sources in the light array. The light arrays are configured as a plurality of transparent tubes with each transparent tube having a plurality of light emitting diodes (LEDs) arranged inside it.

The photo-bioreactor according to the prior art does not provide any heat transfer unit that can help to control the temperature inside the volume. Under these circumstances, the reaction inside the volume will be affected and the result will be an inefficient reaction and possibly a relatively low production rate for the reaction product.

It should be noted that the prior art does not present features that allow heat control inside the reactor. On the contrary, the prior art does not allow the arrangement of a heat transfer unit without compromising the efficiency and the result of the photo-bio reaction.

Other known reactor types comprise a container having a heat transfer jacket or heat transfer coils wrapped around the outside of the container wall to cool down or heat up the contents of the volume of the container. An external feed of cooling water is required

A disadvantage of these types of heat transfer units is that an inhomogeneous heat distribution in the volume may be created, which may result into an irregular reaction (and/or reaction rate) in the volume. By having this type of heat transfer distribution, some parts of the reactor can be overheated, which may cause malfunction of (parts of) the reactor.

Moreover, other disadvantages of the tubular systems relate to relatively high volume occupancy, relatively high demand for illumination and thus relatively high energy consumption, cleaning problems and low efficiency in terms of mass production per unit of volume.

It is therefore an object of the present invention to at least overcome one of the above mentioned disadvantages of the prior art.

It is also an object of the present invention to provide a reactor wherein the rate of the photosynthesis reaction can be optimized based upon the known light response curve (i.e., P/I curve) and in which control of pCO2 (partial pressure of CO₂) can be used for mixing of the reactor contents and for reduction of biofilm growth on the surface of the illumination devices.

### Summary of the invention

The object is achieved by providing a reactor for biochemical processes of the type described above, i.e. a photochemical reaction, wherein the heat transfer unit comprises a plurality of heat pipes, and the light units are mounted on the plurality of heat pipes.

Heat pipes are known in the art for heat transport by means of thermal conduction and phase transitions of the media in the heat pipes.

By having the heat pipes arranged in the volume, i.e., extending into the volume of the tank, and the light units mounted on the heat pipes, a local heat transfer to the outside of the reactor, and a distributed illumination of the reaction fluid contained within the volume is provided. In this manner, a higher efficiency of the reaction process can be achieved, producing a relatively high amount of the reaction product (i.e. biomass of cells or a metabolic endproduct of such cells instead), being this independent of the size of the tank of the reactor.

In a preferred embodiment, the plurality of heat pipes is being vertically positioned, with respect to the longitudinal axis of the tank, in a regular arrangement such that the distance between the longitudinal axis of each heat pipe in each closest neighboring pair is constant.

Such a regular arrangement may in an embodiment provide that at least 80 % of the reactor volume is at least 50 % saturated with light regarding its capacity to carry out oxygenic photosynthesis.

Advantageously, the present invention provides a reactor for photochemical processes that provides an optimal distribution of the illumination of the contents of the volume, while at the same time allows controlling the amount of heat required to optimize the reaction in the volume, i.e., by keeping the temperature in the volume under control at a preferred temperature.

The combination of these effects allows an optimization of a photochemical or photosynthesis-based reaction or a microbiological reaction causing relative improvements in terms of production rates.

According to an embodiment of the invention, the light units comprise a plurality of light emitting diodes (LED) mounted on a printed circuit board (PCB); the printed circuit board being mounted on the heat transfer pipe surface.

In this manner, space occupied by the light units in the volume is minimum, while having LEDs light as a light source reduces the amount of heat produced in the volume. Additionally, this arrangement provides that heat generated by the LEDs can directly be transferred through the heat pipe out of the volume. The energy efficiency of the reactor is thus improved.

Furthermore, due to the relatively long lifetime of LEDs, down-time of the reactor for replacement of light sources can be reduced, and the efficiency of the reactor in terms of costs and production is accordingly improved.

In an embodiment, the light emitting diodes are configured to emit light of either the same or of different emission wavelengths.

In a preferred embodiment of the invention the light units are mounted on the outer circumference of the heat pipes in at last two columns along the length of the heat pipe with an inscribed angle between them. The inscribed angle is 360/n, with n being the number of the columns of light units.

In each columns the light units may be positioned at a same level on the heat pipe. Alternatively, the level of each light unit in each column may differ.

This provides a wide range of possibilities depending on the size or volume of the reactor and also on the distance between each neighboring pair of heat pipes. Therefore, a flexible light configuration is provided to allow adjustment to the requirements of the reaction taking place in the reactor volume.

In an embodiment of the invention, a cover layer is provided that at least covers and insulates the PCB and the LEDs. Optionally, the cover layer also covers the exposed surface area of the heat pipe. The cover layer is transparent and may consist of a resin or plastic encapsulation layer, or a tube.

In this manner, the light units are isolated and protected from the fluid of the volume, providing a longer operating lifetime substantially without compromising the lighting efficiency of the light units or the printed circuit board.

Additionally, the cover layer can provide a thermal isolation between the LEDs and the fluid, substantially preventing the fluid to heat up during illumination.

Moreover, the printed circuit band is mounted onto the heat pipes by means of a bonding agent. This bonding agent can be a heat conductive epoxy comprising a conductive powder in the epoxy. The powder may comprise at least one of copper (alloy), silver (alloy) and aluminum (alloy).

In this manner, a mechanically strong connection is provided by the bonding agent and has excellent thermal and chemical resistance properties.

In an embodiment of the invention, each of the heat pipes has an end extending out the volume of the tank. The extending end of each of the heat pipes is connected to a cooling unit.

In this manner, the heat pipe is arranged to transfer heat from the volume to the cooling unit. During use, at the low end of the heat pipe, the heat pipe is in contact with the relatively warm fluid in the volume. Within the warm section of the heat pipe a liquid cooling medium is heated and will evaporate, forming vapor inside the heat pipe. This vapor will be condensed at the cooler section in contact with the cooling unit. By gravity, the condensed liquid medium will return to the lower end of the heat pipe in the volume of the tank. No external pump to sustain the flows of vapor and liquid within the heat pipe is required. This provides an efficient and continuous heat transfer in the volume of the tank.

In an embodiment of the invention, a distance between the most outer heat pipes in the tank and an inner wall of the tank is about half the distance between each closest neighboring pair of the heat pipes.

In this manner, the illumination as well as the heat transfer of the volume is optimized throughout the volume, providing a substantially similar amount of illumination and heat transfer at every point of the volume.

In an embodiment of the invention, the tank comprises a top cover, and the plurality of heat pipes are mounted in the top cover of the tank, the heat pipes being suspended from the top cover into the tank.

The top cover provides a proper microbiological isolation for the volume, and provides suspension of the heat pipes without the need for any physical connection of the heat pipe(s) with the interior of the tank. This may simplify the maintenance of the reactor since the heat pipes can be reached without the need to open the reactor. The top cover can be arranged to allow a heat pipe to be pulled up from the suspension, without the need to dismantle the reactor.

In an embodiment of the invention, at the lower part of the volume, the tank is provided with a first means for distribution of a first pressurized gas, which in case of a photosynthesis reaction is a gas consisting of CO₂ or enriched in CO₂.

In this manner, a photochemical reaction taking place in a fluid in the volume can be fed with the CO₂ necessary for the reaction. The first means for distribution can be embodied as one or more first nozzles for injecting CO₂ into the volume. In an embodiment the first nozzles can be arranged to enter the volume at a low level, which ensures that CO₂ entering the fluid creates a proper amount of agitation necessary for the reaction fluid, due to the upward flow of the gas through the fluid and prevention of biofilm growth on the surface of the illumination devices.

In an embodiment, such nozzles may comprise membrane contactors, capable of passing CO₂ through the membrane.

Preferably, the first nozzles are positioned in a substantially horizontal plane below the lower ends of the heat pipes at locations corresponding or close to the projected positions of (the ends of) the heat pipes in that plane. In this manner, a flow of CO₂ is created in a region of the reaction fluid close to the heat pipes and the light units mounted thereon where the reaction rate is likely enhanced due to the relatively higher local light intensity. Adding CO₂ in that region typically enhances the photochemical reaction rate in that region.

In a further embodiment of the invention at the lower part of the volume, the tank is provided with a second means (preferably a set of one or more secondary nozzles) for distribution of a second pressurized gas. Primarily, the inlet(s) of the secondary means is/are arranged to enter the volume at a low level, which ensures that due to the upward flow of the second gas through the fluid, a flow in the fluid can be created. Such a flow may enhance the mixing of the reaction fluid and also may enhance an equalization of temperature within the fluid and prevent surface-growth of phototrophic organisms on the illumination devices.

Preferably the second nozzles are positioned in a substantially horizontal plane below the lower ends of the heat pipes at locations in between the projected positions of the heat pipes in that plane. In this manner a gas flow is created in a region of the reaction fluid relatively remote from the heat pipes and light units, which provides agitation and mixing of the reaction fluid in that region with reaction fluid closer to the heat pipes and light units.

Since the purpose for the secondary gas is primarily to provide agitation and mixing, such secondary gas may be an inert gas like N₂, or air.

In an embodiment of the invention, the tank comprises at least one sensor for monitoring a parameter of the reaction fluid in the volume of the tank. The at least one sensor is coupled to a controller device for controlling one or more of the process variables such as light intensity of the light units, flow of the first and/or second gas, cooling capacity of the cooling unit, pH of the reactor fluid, etc.

In an embodiment of the invention, the tank comprises means for capturing at least a portion of the first and second gas flows at the top of volume of the tank.

The captured flows may be returned into the tank as second gas flow for agitation of the fluid. Additionally, in case of a photochemical reaction the CO₂ component of the captured gas can be reintroduced as ingredient for the reaction.

According to an embodiment, the arrangement of heat pipes and light units is designed with a illumination setup of the light units so as to provide that at least 80 % of the reactor volume is at least 50 % saturated with light regarding its capacity to carry out oxygenic photosynthesis, with an upper limit for photo-inhibition less than about 5 %.

In this manner, at least 80 % of the volume of the tank will have a light intensity that saturates photosynthesis for at least 50 %, while simultaneously keeping photo-inhibition to less than about 5 %.

Furthermore, the invention also relates to a use of the reactor as described above in a photochemical reaction, wherein the first means for distribution of a first gas finely disperses a first gas consisting of/enriched in CO₂, and wherein the light units are configured to emit light in a wavelength range of about 500 - about 700 nm.

In an embodiment of this reactor, an arrangement of the heat pipes in the volume with predetermined distance between the heat pipes in a pair of closest neighboring heat pipes in the volume is determined by the absorbance of light of the reaction fluid and the emitted light intensity of the light units.

The distance is chosen in such a manner that within that distance from a light unit at least a lower-limit predetermined reaction efficiency is obtained for a given set of process parameters. The positions of the heat pipes and light units mounted thereon within the arrangement will affect the relative amount of the volume in which this reaction efficiency (or higher) can be achieved.

Advantageous embodiments are further defined by the dependent claims.

### Brief description of the drawings

Other aspects, features and details of the present invention will be readily understood by reference to the following detailed description of preferred embodiments, taken in conjunction with the drawings.

In the appended drawings:
Figure 1 shows a perspective view of the reactor according to an embodiment of the invention;
Figure 2 shows a top view of the reactor of figure 1 without the top cover of the tank;
Figure 3 shows a perspective view of the reactor according to a preferred embodiment of the invention;
Figure 4 shows a perspective view of the heat transfer unit of figure 3;
Figure 5 shows a cross-section of the reactor comprising first means for distribution of a pressurized gas according to a further embodiment of the invention;
Figure 6 shows a cross-section of the reactor comprising a second means for distribution of a pressurized gas according to a further embodiment of the invention.

### Detailed description of the embodiments

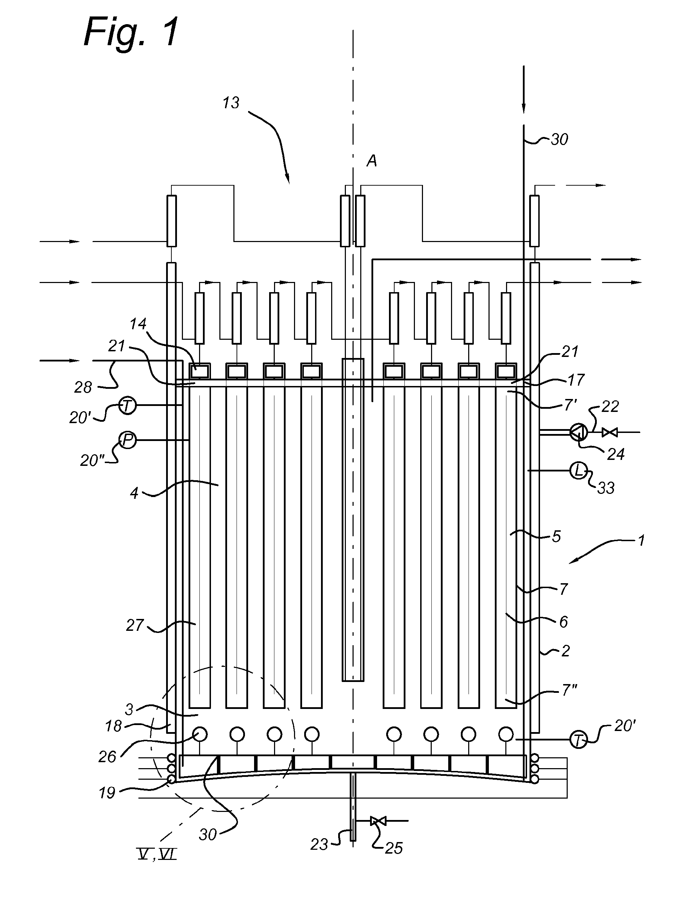

Figure 1 shows a reactor 1 according to the present invention which comprises a tank 2 provided at its top part with a top cover 17. The tank 2 is arranged with a volume 3 that can contain a reaction fluid (e.g. commercial BG-11 medium for the growth of cyanobacteria).

During use, the volume 3 of the reactor 1 contains a reaction fluid 4 in a form of an aqueous solution to cultivate phototrophic microorganisms. These microorganisms use the process of photosynthesis to build their own biomass from light and CO₂ and/or a selected product via molecular engineering of the phototrophic cells..

The reactor further comprises a heat transfer unit 5 with a plurality of heat pipes 7 arranged to extend into the volume 3 and connected to a cooling unit to provide heat transport from the volume to the cooling unit (see below).

According to the invention, along each of the heat pipes, in longitudinal direction, light units consisting of light emitting diodes (LEDs) are mounted. As will be described in more detail below, an interface is provided between the LEDs and the surface of the heat pipe, with the LEDS being mounted on the heat pipe in a manner that allows heat transfer from the LEDs to the heat pipe and hence cooling of the LEDs. Further the LEDs are set up to emit light away from the respective heat pipe on which the LEDs are mounted, into the local volume that surrounds the heat pipe.

As illustrated, the plurality of heat pipes 7 is arranged in the volume 3 of the tank 2. The heat pipes 7 are vertically positioned parallel to the longitudinal axis A of the tank 2. Typically, each of the heat pipes 7 will have the same circumferential diameter. The heat pipes 7 comprise an upper end 7' and a lower end 7". During use, the volume of the tank contains the reaction fluid and the lower end 7" of each of the heat pipes 7 is immersed in the reaction fluid 4 and positioned adjacent to a bottom part of the volume 3. The upper end 7' of each of the heat pipes 7 is situated outside the volume 3 of the tank 2 and extends through the top cover 17 of the tank 2.

A cooling unit 13 is connected to the upper end 7' of the heat pipes 7. The cooling unit 13 provides cooling to the upper end 7' of the heat pipes so as to create a "cold end" of each heat pipe, which creates a location for condensation of the media in the heat pipe and at the same time a thermal gradient between the lower and upper ends of the heat pipe which in combination will create a transfer of heat from the lower end to the upper end. During use, the heat pipes absorb heat from the reaction fluid and heat from the LEDs and transfer this heat to the outside of the reactor.

Optionally, the reactor may be provided with extra heat pipes mounted on, or attached to, the outer wall of the reactor for additional cooling (not shown).

Using LEDs as light units in the reactor reduces the generation of heat within the volume at a given illumination level compared to other light unit types. Moreover, due to the relatively high efficiency of the LEDs a relatively high illumination level can be reached without excessive heat generation.

Further, using the heat pipes provides an efficient transfer of the heat generated by both the reaction fluid and the LEDs, since no external pump is required to circulate the cooling medium in the heat pipes.

In an embodiment, the heat pipes are positioned according to a regular arrangement wherein a distance between each closest neighboring pair of heat pipes 7 is substantially constant. By this arrangement the light units can be distributed in the volume 3 allowing a relatively high illumination level throughout the volume 3. Due to the coupling of heat pipes 7 and LEDs, the cooling of the reaction fluid is distributed correspondingly.

Various arrangements are feasible: for example the heat pipes 7 may be arranged in a square or hexagonal layout in the volume 3.

The tank 2 as here illustrated is transparent for illustrative purposes only. The tank 2 can be made of any suitable material, and can be transparent or opaque for that matter.

In an embodiment, the tank is made of a stainless steel which allows cleaning of a reactor at relatively high temperature.

In an embodiment, to have similar conditions in the reactor close to its inner wall in comparison to the bulk of the volume 3, a distance between the heat pipes 7 positioned next to the inner wall of the tank 2 and the inner wall is about half the distance between heat pipes 7 in each closest neighboring pair.

The tank 2 as illustrated is closeable with the top cover 17. The top cover 17 seals the volume as to maintain an optimal atmosphere inside the volume 3 for the reaction fluid 4. The top cover 17 comprises several openings 21 for allowing the top ends 7' of the heat pipes 7 to extend through the openings 21, allowing easy access to the heat pipes 7 when needed.

At the upper end 7' of the heat pipes 7, a thermal interface (not illustrated) to the cooling unit 13 with the heat pipes 7 is arranged. The skilled in the art will appreciate that various arrangements for a thermal interface are feasible.

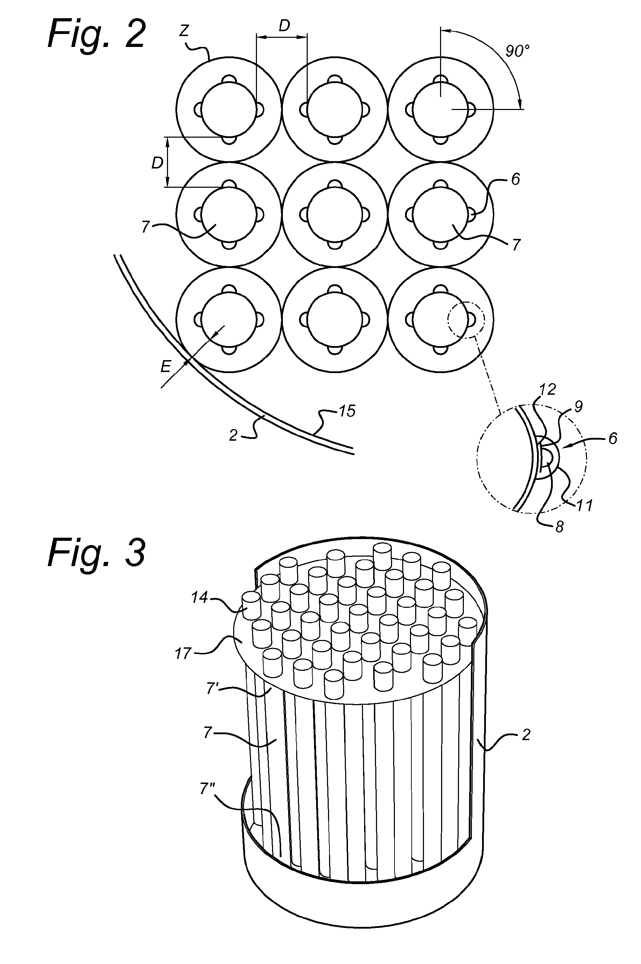

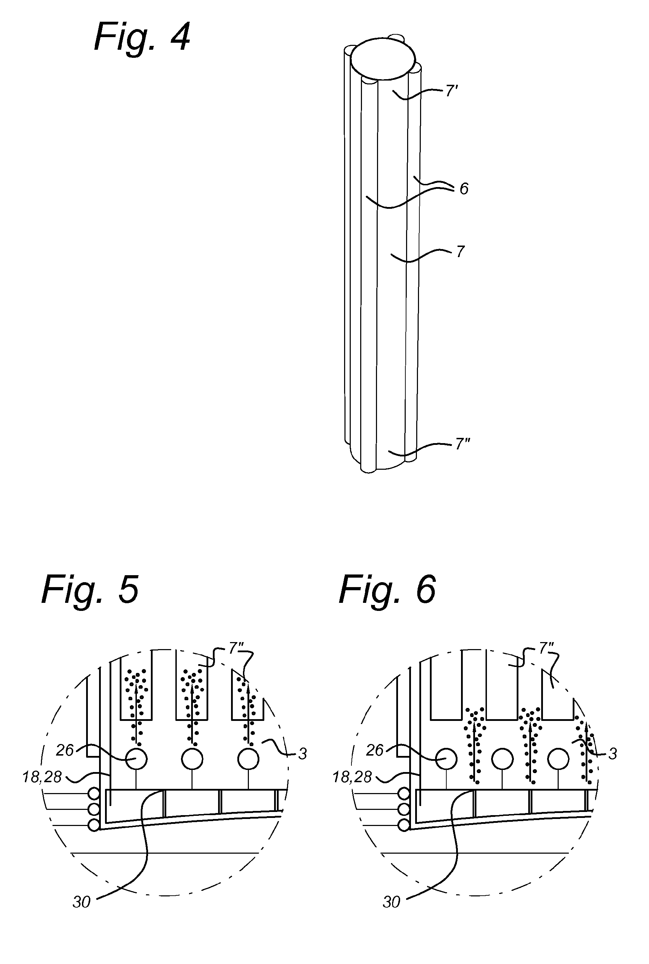

As described above, light units 6 embodied as LEDs are mounted on the outer periphery of each of the heat pipes 7. The light units 6 can be best seen in figures 3 & 4, so it will be described in detailed below.

Additionally, the tank 2 comprises an inlet conduit 22 at the top outer periphery of the tank 2. The inlet conduit 22 is directly connected to a pump 24 for assisting in the filling of the volume 3. The inlet conduit 22 comprises a valve 25 to regulate the inflow of the reaction fluid 4 into the volume 3 of the tank 2. At the bottom of the tank 2, a discharge or outlet conduit 23 is provided, having a valve 25 that opens and closes in order to evacuate the reaction fluid 4 from the volume 3.

As it can be appreciated, at the top side of the tank 2, a pair of control sensors 20 comprising a temperature sensor 20', a pressure sensor 20", and a light sensor 33 are mounted. Preferably, another temperature sensor 20' can be mounted at the bottom part of the tank 2. The control sensors 20 are also connected to a control unit that is arranged to regulate the illumination level of the LEDs and the cooling capacity of the heat pipes. The control sensors are arranged to collect information about the process parameters that the control unit evaluates for controlling the LEDs 6 and the cooling unit 13 /heat pipes 7 as to adapt the reaction efficiency inside the reaction fluid 4 in the volume 3.

Figure 2 illustrates a top view of the tank 2 wherein the top cover 17 has been removed. As illustrated, the plurality of heat pipes 7 are positioned in a regular arrangement (as an example a square arrangement), such that the distances D between closest neighboring heat pipes 7 is constant. Also shown, the closest distance E between heat pipes 7 and the inner wall of the tank 2 is about half the distance D between closest neighboring heat pipes 7.

Around each heat pipe schematically a contour of an illumination zone Z is indicated, in which the illumination level is above a specific threshold of saturation. In an embodiment, the zone Z has an illumination of at least 50 % saturated with light regarding the capacity to carry out oxygenic photosynthesis. Additionally, the zone Z can be further defined to have an upper limit for photo-inhibition less than about 5 %.

It should be noted that the tank 2 as illustrated in figure 2 has a circular cross section, but the invention is not limited to this configuration. Other cross-sections are feasible, for example, elliptical, rectangular, square or hexagonal.

Figures 3 and 4 illustrate an embodiment of the reactor and a single heat pipe 7 in a perspective view respectively. As shown, inside the heat pipe 7 the cooling medium 27 is in a liquid form at the lower warmer section 7" of the heat pipe 7. The liquid evaporates and flows as a vapor in the direction of the illustrated arrows, to a lower partial pressure environment such as the upper colder section 7' of the heat pipe 7. After condensation, the liquid returns to the lower end of the heat pipe.

As shown in figure 4, the LEDs 6 are mounted onto the outer circumference of the heat pipe 7. The LEDs 6 extend along the length B of the heat pipe 7, at least along a section of the heat pipe that is arranged for immersion in the reaction fluid 4, to provide illumination during use to the reaction fluid 4 surrounding the heat pipe 7.

The LEDs 6 are positioned at an equal distance from each other. In the illustrated embodiment, four LEDs are arranged on the circumference of the heat pipe at a same level, i.e., each of the LEDs 6 is positioned at an inscribed angle of 90°, from the neighboring LED.

As best seen in figure 2, the LEDs 6 comprise a light emitting diode (LED) 8 mounted on a printed circuit board (PCB) 9 which may have the form of a strip. The PCB 9 is mounted onto the outer circumference of the heat pipe 7 by means of a bonding agent 12. The bonding agent may be a heat conductive epoxy comprising copper powder in the epoxy, which provides a superior protection from impact and shock, superior conductivity, and also has suitable chemical resistance against the internal environment of the reactor. The LEDs 6 are protected by a transparent cover layer 11 that covers at least the light units 6 (LED and PCB) and protects the light units 6 from the chemical solution 4 or corrosive agents, while allowing the light emitted by the LED 8 to reach all areas of the volume 3.

The cover layer 11 as illustrated may be embodied as a resin or plastic encapsulation layer or tube.

In case of a tube, the cover layer 11 covers the LEDs 6 and the heat pipe 7 on which the LEDs are mounted in its entirety.

The LEDs 6 are connected to the controller device (not illustrated) that is arranged in connection with the control sensors to determine and control the light transmission between the LEDs 6 to obtain suitable illumination conditions. In an embodiment, the controller device is arranged with the capability to adjust the operating frequency of the LEDs 8 in relation to requirements of the chemical or biological process taking place in the volume 3.

Figure 5 illustrates a further embodiment of the present invention wherein the tank 2 comprises at the lower part of the volume 3 a first means for distribution of a first pressurized gas 18. In case of a photosynthesis reaction such a gas consists of or is enriched with CO₂. The first means for distribution of a first pressurized gas 18 as illustrated have the form a plurality of first nozzles 26 from which the CO₂ is injected into the reaction fluid 4 contained in the volume 3. The plurality of first nozzles 26 are distributed in such a way that sufficient CO₂ gas is provided in the reaction fluid 4, and in such a manner that the CO₂ gas is provided around the entire periphery of the heat pipes 7.

The first nozzles 26 can be arranged to enter the volume at a low level, which ensures that CO₂ entering the fluid creates a proper amount of agitation necessary for the reaction fluid due to the upward flow of the gas through the fluid.

Preferably, the first nozzles are positioned in a substantially horizontal bottom plane below the lower ends of the heat pipes at locations corresponding with or close to the projected positions of (the ends of) the heat pipes in that plane. In this manner, a flow of CO₂ is created in a region of the reaction fluid close to the periphery of the heat pipes and the light units mounted.

By positioning a nozzle under a heat pipe, advantageously it is achieved that growth of the phototropic microorganism on surface or wall is reduced or prevented.

In an embodiment the heat pipes are each inserted in a transparent tube, which has a convex bottom end which enhances the distribution of CO₂ around the tube.

The transparent tube may be formed from any suitable material. In an embodiment, polysulfon is used which has no interaction with the fluid and is substantially insusceptible to fouling.

It should be noted that it is possible to capture and recycle a portion of the CO₂ gas escaping from the top of the basin to further exhaust the CO₂ gas, by recycle means (not illustrated) installed at the top cover of the volume 3.

As shown, an inlet duct 28 for the CO₂ comprises a valve 29 as to regulate the inflow of CO₂ entering the volume 3. The valve 29 can be connected to the controller device (not illustrated) as to regulate the inflow of CO₂ injected into the volume 3.

The reactor 1 may comprise a control system equipped with sensors for sensing presence and levels of the gas (gases) such as CO₂. The control system is arranged for controlling the partial gas pressures in the liquid and gas phase in the reactor volume.

Figure 6 shows a further embodiment of the invention wherein the tank 2 further comprises a second means for distribution of a secondary pressurized gas 19 into the volume 3. The second means for distribution of a secondary pressurized gas 19 as illustrated comprises a plurality of second nozzles 32 that inject the secondary gas into the reaction fluid 4 contained in the volume 3. According to an embodiment, the second nozzles are positioned at a bottom plane of the volume at locations below the lower ends of the heat pipes and in between the projected positions of the heat pipes on the bottom plane. In this manner, a gas flow from the second nozzles will create an agitation and upward flow of the reaction fluid in between the heat pipes. For this purpose the secondary gas may be an inert gas, like N₂, or air.

The gas flow from the second nozzles can assist in the reduction or prevention of growth of the phototropic microorganism on the surfaces or walls of the heat pipes.

An inlet duct 30 for the secondary gas is provided, having a valve 31 to regulate the inflow of the secondary gas into the volume 3. The valve 31 can also be connected to the controller device (not illustrated) as to regulate the inflow of secondary gas injected into the volume 3.

The first and second means for distribution of a pressurized gas 18, 19 are installed in an arrangement that the nozzles 26, 32 are positioned at a distance from each other, as to provide the CO₂ and the N₂ at different locations in the volume 3.

The present invention can further be characterized by the following embodiments:
Embodiment 1. A reactor for biochemical and/or photochemical processes comprising:
   - a tank having a volume for containing a reaction fluid,
   - a heat transfer unit being arranged in the volume,
   - a plurality of light units arranged in the volume of the tank,
   wherein the heat transfer unit comprises a plurality of heat pipes, and the light units are mounted on the plurality of heat pipes.
Embodiment 2. The reactor according to embodiment 1, wherein the plurality of heat pipes are vertically positioned, with respect to the longitudinal axis of the tank, in a regular arrangement such that the distance between the longitudinal axis of heat pipes in each closest neighboring pair of heat pipes is constant.
Embodiment 3. The reactor according to any one of the embodiments 1 or 2, wherein the light units comprise a plurality of light emitting diodes (LED) mounted on a printed circuit board, the printed circuit board being mounted on the heat pipe surface.
Embodiment 4. The reactor according to any one of the embodiments 1 - 3, wherein the light units are mounted on the outer circumference of the heat pipes in at last two columns along the length of the heat pipe with an inscribed angle between them.
Embodiment 5. The reactor according to any one of the embodiments 1 - 4, wherein the inscribed angle is 360/n, with n being the number of columns of light units.
Embodiment 6. The reactor according to any one of the preceding embodiments, wherein a transparent cover layer is provided that at least covers and insulates the PCB and the LEDs.
Embodiment 7. The reactor according to embodiment 6, wherein the cover layer additionally covers a surface area of the heat pipe exposed to the volume.
8. The reactor according to any one of the preceding embodiments, wherein the cover Embodiment layer comprises a resin or a plastic encapsulation layer or tube.
Embodiment 9. The reactor according to any one of embodiments 6 - 8, wherein the cover layer is made from a polysulfon-based material.
Embodiment 10. The reactor according to any one of the preceding embodiments, wherein a bottom end of the heat pipe has a convex rounding.
Embodiment 11. The reactor according to any one of the preceding embodiments, wherein each of the heat pipes has an end extending out the volume of the tank; and wherein the extending end of each of the heat pipes is connected to a cooling unit.
Embodiment 12. The reactor according to any one of the preceding embodiments, wherein the distance between the heat pipes and an inner wall of the tank is about half the distance between the each closest neighboring pair of the heat pipes.
Embodiment 13. The reactor according to any one of the preceding embodiments, wherein the tank comprises a top cover, and the plurality of heat pipes are mounted in the top cover of the tank, the heat pipes being suspended from the top cover into the tank.
Embodiment 14. The reactor according to any one of the preceding embodiments, wherein at the lower part of the volume, the tank is provided with a first means for distribution of a first pressurized gas, preferably CO₂.
Embodiment 15. The reactor according to embodiment 13 or 14, wherein the first means comprises a plurality of first nozzles, the first nozzles are positioned in a substantially horizontal plane below the lower ends of the heat pipes at locations corresponding to or close to projected positions of the heat pipes or the ends thereof in that plane.
Embodiment 16. The reactor according to embodiment 15, wherein one or more of the first nozzles are embodied as a membrane contactor, capable of passing the first pressurized gas through the membrane.
Embodiment 17. The reactor according to any one of the preceding embodiments 14 - 16, wherein the first pressurized gas is a gas consisting of CO₂ or enriched in CO₂.
Embodiment 18. The reactor according to any one of the preceding embodiments, wherein at the lower part of the volume, the tank is provided with a second means for distribution of a second pressurized gas.
Embodiment 19. The reactor according to embodiment 18, wherein the second pressurized gas is an inert gas like N₂, or air.
Embodiment 20. The reactor according to embodiment 18 or 19, wherein the second means comprises a plurality of second nozzles, the second nozzles are positioned in a substantially horizontal plane below the lower ends of the heat pipes at locations under or inbetween projected positions of the heat pipes in that plane.
Embodiment 21. The reactor according to any one of the preceding embodiments, wherein the tank comprises at least one sensor for monitoring the reaction fluid in the volume of the tank, the at least one sensor being coupled to a controller device for controlling one or more of the process variables such as light intensity of the light units, flow of the first and/or second pressurized gas, cooling capacity of the cooling unit.
Embodiment 22. The reactor according to any one of the preceding embodiments, wherein the tank comprises means for capturing and recycling at least a portion of the first and/or second pressurized gas at a top of the volume of the tank.
Embodiment 23. The reactor according to any one of the preceding embodiments, wherein the reactor comprises secondary heat pipes each attached either in its top cover or at its outer wall for transfer of heat from the reactor wall.
Embodiment 24. The reactor according to any one of the preceding embodiments, having a illumination setup of the light units so as to provide that at least 80 % of the reactor volume is at least 50 % saturated with light regarding its capacity to carry out oxygenic photosynthesis, with an upper limit for photo-inhibition less than about 5 %.
Embodiment 25. Use of the reactor according to any one of the preceding embodiments 14 - 24, in a photochemical reaction in a reaction fluid, wherein the first means for distribution of a first pressurized gas finely disperses a first gas consisting of/enriched in CO₂, in said fluid and wherein the light units are configured to emit light in a wavelength range of about 500 - about 700 nm, the emitted light being either of a polychromatic or of a monochromatic nature.

It will be apparent to the person skilled in the art that other embodiments of the invention can be conceived and reduced to practice without departing from the true spirit of the invention, the scope of the invention being limited only by the appended claims. The above description is not intended to limit the invention.

### Reference numerals

- 1.: Reactor
- 2.: Tank
- 3.: Volume
- 4.: Reaction fluid
- 5.: Heat transfer unit
- 6.: Light unit (LED)
- 7.: Heat pipe
- 7'.: Top end of heat pipe
- 7".: Lower end of heat pipe
- 8.: Light emitting diode unit (LED unit)
- 9.: Printed circuit board (PCB)
- 10.: Inscribed angle
- 11.: Cover layer
- 12.: Bonding agent
- 13.: Cooling unit
- 14.: Extending end of heat pipe
- 15.: Inner wall of the tank
- 16.: Longitudinal axis of the tank
- 17.: Top cover of the tank
- 18.: First means for distribution of a first pressurized gas
- 19.: Second means for distribution of a second pressurized gas
- 20.: Control sensor
- 20'.: Temperature sensor
- 20".: Pressure sensor
- 21.: Openings of the top cover of the tank
- 22.: Inlet conduit
- 23.: Discharge conduit
- 24.: Pump
- 25.: Valve
- 26.: first (CO₂) nozzle
- 27.: Heat pipe cooling media
- 28.: Inlet duct for CO₂
- 29.: Valve of the inlet duct for CO₂
- 30.: Inlet duct for N2
- 31.: Valve of the inlet duct for N₂
- 32.: second (N₂) nozzle
- 33.: Light sensor
- D.: Distance between neighboring pair of heat pipes
- E.: Distance between the inner wall of the tank and a neighboring heat pipe

## Claims

1. A reactor for biochemical and/or photochemical processes comprising:
- a tank having a volume for containing a reaction fluid,
- a heat transfer unit being arranged in the volume,
- a plurality of light units arranged in the volume of the tank,
wherein the heat transfer unit comprises a plurality of heat pipes, and the light units are mounted on the plurality of heat pipes.

2. The reactor according to claim 1, wherein the plurality of heat pipes are vertically positioned, with respect to the longitudinal axis of the tank, in a regular arrangement such that the distance between the longitudinal axis of heat pipes in each closest neighboring pair of heat pipes is constant.

3. The reactor according to any one of the claims 1 or 2, wherein the light units comprise a plurality of light emitting diodes (LED) mounted on a printed circuit board, the printed circuit board being mounted on the heat pipe surface.

4. The reactor according to any one of the claims 1 - 3, wherein the light units are mounted on the outer circumference of the heat pipes in at last two columns along the length of the heat pipe with an inscribed angle between them, wherein the inscribed angle is 360/n, with n being the number of columns of light units.

5. The reactor according to any one of the preceding claims, wherein a transparent cover layer is provided that at least covers and insulates the PCB and the LEDs.

6. The reactor according to claim 5, wherein the cover layer is made from a polysulfon-based material.

7. The reactor according to any one of the preceding claims, wherein each of the heat pipes has an end extending out the volume of the tank; and wherein the extending end of each of the heat pipes is connected to a cooling unit.

8. The reactor according to any one of the preceding claims, wherein the distance between the heat pipes and an inner wall of the tank is about half the distance between the each closest neighboring pair of the heat pipes.

9. The reactor according to any one of the preceding claims, wherein the tank comprises a top cover, and the plurality of heat pipes are mounted in the top cover of the tank, the heat pipes being suspended from the top cover into the tank.

10. The reactor according to any one of the preceding claims, wherein at the lower part of the volume, the tank is provided with a first means for distribution of a first pressurized gas, preferably CO₂, and the first means comprises a plurality of first nozzles, the first nozzles are positioned in a substantially horizontal plane below the lower ends of the heat pipes at locations corresponding to or close to projected positions of the heat pipes or the ends thereof in that plane.

11. The reactor according to claim 10, wherein one or more of the first nozzles are embodied as a membrane contactor, capable of passing the first pressurized gas through the membrane.

12. The reactor according to any one of the preceding claims, wherein at the lower part of the volume, the tank is provided with a second means for distribution of a second pressurized gas the second pressurized gas being an inert gas like N₂, or air, and
the second means comprising a plurality of second nozzles, the second nozzles being positioned in a substantially horizontal plane below the lower ends of the heat pipes at locations under or inbetween projected positions of the heat pipes in that plane.

13. The reactor according to any one of the preceding claims, wherein the tank comprises means for capturing and recycling at least a portion of the first and/or second pressurized gas at a top of the volume of the tank.

14. The reactor according to any one of the preceding claims, having a illumination setup of the light units so as to provide that at least 80 % of the reactor volume is at least 50 % saturated with light regarding its capacity to carry out oxygenic photosynthesis, with an upper limit for photo-inhibition less than about 5 %.

15. Use of the reactor according to any one of the preceding claim 10 - 14, in a photochemical reaction in a reaction fluid, wherein the first means for distribution of a first pressurized gas finely disperses a first gas consisting of/enriched in CO₂, in said fluid and wherein the light units are configured to emit light in a wavelength range of about 500 - about 700 nm, the emitted light being either of a polychromatic or of a monochromatic nature.
